# EUROPEAN PATENT APPLICATION

(11) **EP 2 719 684 A1**
(43) Date of publication of application: **16.04.2014**
(21) Application number: 12793853.8
(22) Date of filing: 30.05.2012
(51) Int. Cl.: C07C 271/12, C07C 233/31, C07D 261/04, C07B 61/00

(54) **METHOD OF MANUFACTURING BENZYLAMINE COMPOUND**

(30) Priority: 03.06.2011 JP 2011125013; 17.06.2011 JP 2011134837; 31.01.2012 JP 2012017674
(71) Applicant: Sumitomo Chemical Company, Limited, Tokyo 104-8260 (JP)
(72) Inventor: KUMAMOTO, Koji, Takarazuka-shi Hyogo 665-8555 (JP); UJIHARA, Kazuya, Takarazuka-shi Hyogo 665-8555 (JP)
(74) Representative: Vossius & Partner
(86) International application number: PCT/JP2012/064488
(87) International publication number: WO 2012/165651

(57) **Abstract**

A new method for producing a benzylamine compound represented by formula (7) : 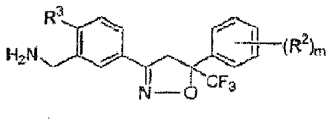 [wherein R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)],
and an intermediate for the said methodare provided.

## Description

### TECHNICAL FIELD

The present invention relates to a method for producing a benzylamine compound and a production intermediate thereof.

### BACKGROUND ART

It has been known that a benzylamine compound represented by the following formula (7) is useful, for example, as an active ingredient of a pesticide. In addition, for example, WO2010/032437 discloses a method for synthesizing a benzylamine compound represented by the following formula (7).

### DISCLOSURE OF THE INVENTION

The above conventional method is not necessarily satisfactory in the production of the benzylamine compound represented by the following formula (7).

Thus, an object of the present invention is to provide a new method that can produce the benzylamine compound represented by the following formula (7) and a production intermediate thereof.

As a result of intensive studies, the present inventors have found that a benzylamine compound represented by the following formula (7) can be produced by reacting an amide compound represented by the following formula (1) with a trifluoroacetophenone compound represented by the following formula a (2) to produce an aldol compound represented by the following formula (3),
reacting the obtained aldol compound represented by the following formula (3) with an acid anhydride compound represented by the following formula (4) to produce an enone compound represented by the following formula (5), reacting the obtained enone compound represented by the following formula (5) with hydroxylamine to produce an isoxazoline compound represented by the following formula (6), and
reacting the obtained isoxazoline compound represented by the following formula (6) with an acid.

More specifically, the present invention is as described below.
[1] An aldol compound represented by formula (3): 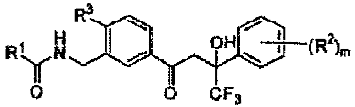 [wherein R¹ represents a C1-C12 alkyl group optionally having one or more halogen atoms, a C3-C12 cycloalkyl group optionally having one or more halogen atoms, or a C1-C12 alkoxy group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)].
[2] An enone compound represented by formula (5): 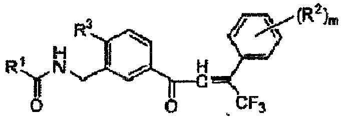 [wherein R¹ represents a C1-C12 alkyl group optionally having one or more halogen atoms, a C3-C12 cycloalkyl group optionally having one or more halogen atoms, or a C1-C12 alkoxy group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)].
[3] tert-Butyl (5-acetyl-2-chlorobenzyl)carbamate.
[4] tert-Butyl {2-chloro-5-[5-(3,5-dichlorophenyl)-5-trifluoromethyl-4,5-dihydroisoxazol-3-yl]benzyl}carbamate.
[5] A method for producing an aldol compound represented by formula (3) : 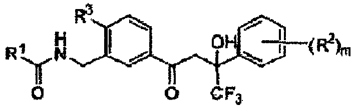 [wherein R¹ represents a C1-C12 alkyl group optionally having one or more halogen atoms, a C3-C12 cycloalkyl group optionally having one or more halogen atoms or a C1-C12 alkoxy group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)] (hereinafter referred to as aldol compound (3)),
   comprising a step of reacting an amide compound represented by formula (1) : 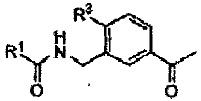 [wherein R¹ represents a C1-C12 alkyl group optionally having one or more halogen atoms, a C3-C12 cycloalkyl group optionally having one or more halogen atoms or a C1-C12 alkoxy group optionally having one or more halogen atoms, and R³ represents a halogen atom or a hydrogen atom (hereinafter referred to as amide compound (1))
   with a trifluoroacetophenone compound represented by formula (2) : 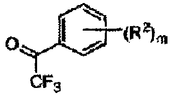 [wherein R² represents a C1-C6 alkyl group optionally having one or more halogen atoms or a halogen atom, and m represents an integer of 0 to 5 (when m is an integer of 2 to 5, R²s may be different from one another)] (hereinafter referred to as trifluoroacetophenone compound (2)).
[6] A method for producing an enone compound represented by formula (5) : 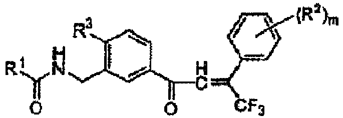 [wherein R¹ represents a C1-C12 alkyl group optionally having one or more halogen atoms, a C3-C12 cycloalkyl group optionally having one or more halogen atoms, or a C1-C12 alkoxy group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)] (hereinafter referred to as enone compound (5)),
   comprising a step of reacting the aldol compound (3) with an acid anhydride compound represented by formula (4): 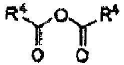 [wherein R⁴ represents a C1-C12 alkyl group optionally having one or more halogen atoms or a C1-C12 alkoxy group optionally having one or more halogen atoms] (hereinafter referred to as acid anhydride compound (4)).
[7] A method for producing an isoxazoline compound represented by formula (6): 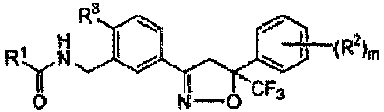 [wherein R¹ represents a C1-C12 alkyl group optionally having one or more halogen atoms, a C3-C12 cycloalkyl group optionally having one or more halogen atoms, or a C1-C12 alkoxy group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)] (hereinafter referred to as isoxazoline compound (6)),
   comprising a step of reacting the enone compound (5) with hydroxylamine.
[8] A method for producing a benzylamine compound represented by formula (7): 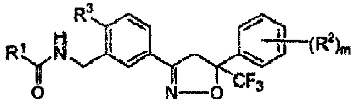 [wherein R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)] (hereinafter referred to as benzylamine compound (7)),
   comprising a step of reacting the the isoxazoline compound (6) with an acid.
[9] A method for producing the benzylamine compound (7), comprising steps of reacting the amide compound (1) with the trifluoroacetophenone compound (2) to produce the aldol compound (3) ; reacting the obtained aldol compound (3) with the acid anhydride compound (4) to produce the enone compound (5); reacting the obtained enone compound (5) with hydroxylamine to produce the isoxazoline compound (6); and reacting the obtained isoxazoline compound (6) with an acid.

### EFFECT OF THE INVENTION

According to the present invention, a new method for producing the benzylamine compound (7), a production intermediate thereof and the like can be provided.

### MODE FOR CURRYING OUT THE INVENTION

Examples of the "C1-C12 alkyl group optionally having one or more halogen atoms" represented by R¹ include a methyl group, an ethyl group, a propyl group, an isopropyl group, a butyl group, a tert-butyl group, an isobutyl group, a pentyl group, a hexyl group, a dodecyl group, a trifluoromethyl group, and a 3,3,3-trifluoropropyl group.

Examples of the "C3-C12 cycloalkyl group optionally having one or more halogen atoms" represented by R¹ include a cyclopropyl group, a cyclobutyl group, a cyclopentyl group, a cyclohexyl group, and a cycloheptyl group.

Examples of the "C1-C12 alkoxy group optionally having one or more halogen atoms" represented by R¹ include a methoxy group, an ethoxy group, a propoxy group, an isopropoxy group, and a tert-butoxy group.

Examples of the "halogen atom" represented by R² include a fluorine atom, a chlorine atom, a bromine atom, and an iodine atom.

Examples of the "C1-C6 alkyl group optionally having one or more halogen atoms" represented by R² include a methyl group and a trifluoromethyl group.

Examples of the "halogen atom" represented by R³ include a fluorine atom, a chlorine atom, a bromine atom, and an iodine atom.

Examples of the "C1-C12 alky- group optionally having one or more halogen atoms" represented by R⁴ include a methyl group and a trifluoromethyl group.

Examples of the "C1-C12 alkoxy group optionally having one or more halogen atoms" represented by R⁴ include a methoxy group and a tert-butoxy group.

In the production of the aldol compound (3) from the amide compound (1) and the trifluoroacetophenone compound (2), the aldol compound (3) is produced by reacting the amide compound (1) with the trifluoroacetophenone compound (2).

The amount of the trifluoroacetophenone compound (2) used for the reaction is usually 0.1 to 10 mol, based on 1 mol of the amide compound (1).

The reaction is usually carried out in the presence of a base.

Examples of the base used for the reaction include organic bases such as triethylamine and diazabicycloundecene (1,8-diazabicyclo[5.4.0]-7-undecene). These bases can be used alone or in combination of two or more thereof.

The amount of the base used for the reaction is usually 0.1 to 10 mol, based on 1 mol of the amide compound (1).

The reaction is usually carried out in the presence of a solvent. Examples of the solvent used in the reaction include ethers such as tetrahydrofuran and hydrocarbons such as toluene, and these solvents can be used alone or in combination of two or more thereof.

The amount of the solvent used when the reaction is carried out in a solvent is 100 L or less, based on 1 mol of the amide compound (1).

The reaction temperature is usually in the range of -20 to 200°C or not more than the boiling point of the solvent used in the reaction.

The reaction time is usually in the range of 0.1 to 100 hours.

As mentioned above, the aldol compound (3) can be produced. Further, it is also possible to purify the obtained aldol compound by means such as extraction by separation, filtration, recrystallization, or column chromatography, as necessary.

In the production of the enone compound (5) from the aldol compound (3), the enone compound (5) is produced by reacting the aldol compound (3) with the acid anhydride compound (4).

The amount of the acid anhydride compound (4) used for the reaction is usually 1 to 10 mol, based on 1 mol of the aldol compound (3).

The reaction is usually carried out in the presence of a base.

Examples of the base used for the reaction include organic bases such as triethylamine, pyridine, and N,N-dimethyl-4-aminopyridine. These bases can be used alone or in combination of two or more thereof.

The amount of the base used for the reaction is usually 0.1 to 10 mol, based on 1 mol of the aldol compound (3).

The reaction is usually carried out in the presence of a solvent. Examples of the solvent used for the reaction include ethers such as tetrahydrofuran, hydrocarbons such as toluene, and halogenated hydrocarbons such as chloroform, and these solvents can be used alone or in combination of two or more thereof.

The amount of the solvent used when the reaction is carried out in a solvent is 100 L or less, based on 1 mol of the aldol compound (3).

The reaction temperature is usually in the range of -20 to 200°C or not more than the boiling point of the solvent used in the reaction.

The reaction time is usually in the range of 0.1 to 100 hours.

After carrying out the reaction, the resultant can also be reacted with sodium methoxide. Examples of the solvent used in the step include methanol.

As mentioned above, the enone compound (5) can be produced. Further, it is also possible to purify the obtained enone compound by means such as extraction by separation, filtration, recrystallization, or column chromatography, as necessary.

In the production of the isoxazoline compound (6) from the enone compound (5), the isoxazoline compound (6) is produced by reacting the enone compound (5) with hydroxylamine.

The reaction is usually carried out in the presence of a base.

Examples of the base used for the reaction include metal hydroxides such as sodium hydroxide and organic bases such as diazabicycloundecene. These bases can be used alone or in combination of two or more thereof.

The amount of the base used for the reaction is usually 0.01 to 10 mol, based on 1 mol of the enone compound (5).

The reaction is usually carried out in the presence of a solvent. Examples of the solvent used for the reaction include hydrocarbons such as toluene, ethers such as methyl tert-butyl ether, and water, and these solvents can be used alone or in combination of two or more thereof.

The amount of the solvent used when the reaction is carried out in a solvent is 100 L or less, based on 1 mol of the enone compound (5).

As the hydroxylamine used for the reaction, hydroxylamine in the form of an aqueous solution or a salt such as hydroxylamine hydrochloride can be used. These can be used alone or in combination of two or more thereof.

The amount of the hydroxylamine used for the reaction is usually 1 to 10 mol, based on 1 mol of the enone compound (5).

The reaction can be carried out also in the presence of a phase transfer catalyst. Examples of the phase transfer catalyst used for the reaction include tetrabutylammonium bromide and tetraoctylammonium chloride. These phase transfer catalysts can be used alone or in combination of two or more thereof.

The amount of the phase transfer catalyst used for the reaction is usually 0.001 to 1 mol, based on 1 mol of the enone compound (5).

The reaction temperature is usually in the range of -20 to 200°C or the boiling point of the solvent used in the reaction.

The reaction time is usually in the range of 0.1 to 100 hours.

As mentioned above, the isoxazoline compound (6) can be produced. Further, it is also possible to purify the obtained isoxazoline compound by means such as extraction by separation, filtration, recrystallization, or column chromatography, as necessary.

In the production of the benzylamine compound (7) from the isoxazoline compound (6) and an acid, the benzylamine compound (7) is produced by reacting the isoxazoline compound (6) with the acid.

The amount of the acid used in the reaction is usually 0.1 to 100 mol, based on 1 mol of the isoxazoline compound (6).

Examples of the acid used fcr the reaction include carboxylic acids such as trifluoroacetic acid and mineral acids such as hydrochloric acid. These acids can be used alone or in combination of two or more thereof.

The reaction can be carried out also in the presence of a solvent. Examples of the solvent used for the reaction include ethanol.

The reaction temperature is usually in the range of -20 to 200°C or the boiling point of the acid used in the reaction.

The reaction time is usually in the range of 0.1 to 100 hours.

As mentioned above, the benzylamine compound (7) can be produced. Further, it is also possible to purify the obtained benzylamine compound by means such as extraction by separation, filtration, recrystallization, or column chromatography, as necessary.

The amide compound (1) can be produced by the method described in WO2004/058681, WO2008/134036, or Journal of Labelled compounds and Radiopharmaceuticals, vol. 51, p. 72 (2008).

Some of the trifluoroacetophenone compounds (2) are commercially available, and other compounds can be produced, for example, according to the method described in Canadian Journal of Chemistry vol. 58, p. 2491 (1980) or Angewandte Chemie International Edition vol. 37, p- 820 (1998).

Specific examples of the aldol compound (3) are shown below.

Aldol compounds represented by formula (3): 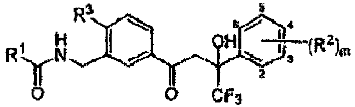 [wherein R¹, (R²)ₘ, and R³ represent the combination described in Table 1 below].

**[Table 1]**

| No. | R¹ | (R²)ₘ | R³ |
|---|---|---|---|
| 1 | CH₃ | 3-Cl, 5-Cl | Cl |
| 2 | CH₂CH₃ | 3-Cl, 5-Cl | Cl |
| 3 | CH₂CH₂CH₃ | 3-Cl, 5-Cl | Cl |
| 4 | CH(CH₃)₂ | 3-Cl, 5-Cl | Cl |
| 5 | C(CH₃)₃ | 3-Cl, 5-Cl | Cl |
| 6 | CH₂CH(CH_{3 2} | 3-Cl, 5-Cl | Cl |
| 7 | CF₃ | 3-Cl, 5-Cl | Cl |
| 8 | CH₂CH₂CF₃ | 3-Cl, 5-Cl | Cl |
| 9 | OCH₃ | 3-Cl, 5-Cl | Cl |
| 10 | OCH₂CH₃ | 3-Cl, 5-Cl | Cl |
| 11 | OCH(CH₃)₂ | 3-Cl, 5-Cl | Cl |
| 12 | OC(CH₂)₃ | 3-Cl, 5-Cl | Cl |
| 13 | CH₃ | 3-Cl, 5-Cl | H |
| 14 | CH₃ | 3-Cl, 5-Cl | F |
| 15 | CH₃ | 3-Cl, 5-Cl | Br |
| 16 | Cyclopropyl | 3-Cl, 5-Cl | Cl |
| 17 | Cyclobutyl | 3-Cl, 5-Cl | Cl |
| 18 | Cyclopentyl | 3-Cl, 5-Cl | Cl |
| 19 | Cyclohexyl | 3-Cl, 5-Cl | Cl |
| 20 | Cycloheptyl | 3-Cl, 5-Cl | Cl |
| 21 | (CH₂)₃CH₃ | 3-Cl, 5-Cl | Cl |
| 22 | (CH₂)₄CH₃ | 3-Cl, 5-Cl | Cl |
| 23 | (CH₂)₅CH₃ | 3-Cl, 5-Cl | Cl |
| 24 | (CH₂)₁₁CH₃ | 3-Cl, 5-Cl | Cl |
| 25 | CH₃ | 3-CF₃, 5-CF₃ | Cl |
| 26 | CH₃ | 3-Cl, 4-Cl, 5-Cl | Cl |

Specific examples of the enone compound (5) are shown below.

Enone compounds represented by formula (5) : 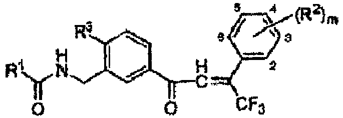 [wherein R¹, (R²) m, and R³ represent the combination described in Table 2 below].

**[Table 2]**

| No. | R¹ | (R²)m | R³ |
|---|---|---|---|
| 1 | CH₃ | 3-Cl, 5-Cl | Cl |
| 2 | CH₂CH₃ | 3-Cl, 5-Cl | Cl |
| 3 | CH₂CH₂CH₃ | 3-Cl, 5-Cl | Cl |
| 4 | CH(CH₃)₂ | 3-Cl, 5-Cl | Cl |
| 5 | C(CH₃)₃ | 3-Cl, 5-Cl | Cl |
| 6 | CH₂CH(CH₃)₂ | 3-Cl, 5-Cl | Cl |
| 7 | CF₃ | 3-Cl, 5-Cl | Cl |
| 8 | CH₂CH₂CF₃ | 3-Cl, 5-Cl | Cl |
| 9 | OCH₃ | 3-Cl, 5-Cl | Cl |
| 10 | OCH₂CH₃ | 3-Cl, 5-Cl | Cl |
| 11 | OCH(CH₃)₂ | 3-Cl, 5-Cl | Cl |
| 12 | OC(CH₃)₃ | 3-Cl, 5-Cl | Cl |
| 13 | CH₃ | 3-Cl, 5-Cl | H |
| 14 | CH₃ | 3-Cl, 5-Cl | F |
| 15 | CH₃ | 3-Cl, 5-Cl | Br |
| 16 | Cyclopropyl | 3-Cl, 5-Cl | Cl |
| 17 | Cyclobutyl | 3-Cl, 5-Cl | Cl |
| 18 | Cyclopentyl | 3-Cl, 5-Cl | Cl |
| 19 | Cyclohexyl | 3-Cl, 5-Cl | Cl |
| 20 | Cycloheptyl | 3-Cl, 5-Cl | Cl |
| 21 | (CH₂)₃CH₃ | 3-Cl, 5-Cl | Cl |
| 22 | (CH₂)₄CH₃ | 3-Cl, 5-Cl | Cl |
| 23 | (CH₂)₅CH₃ | 3-Cl, 5-Cl | Cl |
| 24 | (CH₂)₁₁CH₃ | 3-Cl, 5-Cl | Cl |
| 25 | CH₃ | 3-CF₃, 5-CF₃ | Cl |
| 26 | CH₃ | 3-Cl, 4-Cl, 5-Cl | Cl |

Here, in (R²)ₘ of Table 1 and Table 2 above, for example, the description of "3-Cl, 5-Cl" means that (R²)ₘ is substituents in the positions 3 and 5, m is 2, and R²s are each a chlorine atom.

### EXAMPLES

Examples of the present invention are shown below, but the present invention is not limited by these examples.

### Example 1

### Production of tert-butyl (5-acetyl-2-chlorobenzyl)carbamate

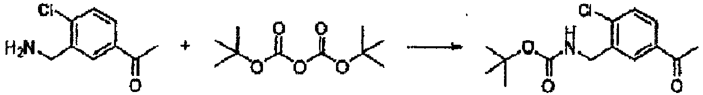

In tetrahydrofuran (100 ml) was dissolved 1-(3-aminomethyl-4-chlorophenyl)ethanone (13.58 g), and di-tert-butyl dicarbonate (15.90 g) was added thereto at room temperature. After stirring at room temperature for 1 hour, this reaction mixture liquid was concentrated under reduced pressure, and the resulting residue was dissolved in methyl tert-butyl ether, and then washed with 1 M hydrochloric acid and an aqueous saturated sodium bicarbonate solution. The organic layer was dried over anhydrous sodium sulfate and then concentrated under reduced pressure. The resulting residue was subjected to silica gel column chromatography to obtain tert-butyl (5-acetyl-2-chlorobenzyl)carbamate (16.31 g). ¹H-NMR (CDCl₃) δ: 7.95 (1H, d), 7.79 (1H, dd), 7.44 (1H, d), 5.15 (1H, br s), 4.44 (2H, d), 2.58 (3H, s), 1.46 (9H, s).

### Example 2

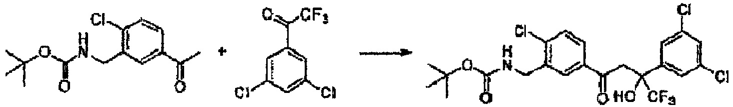

### Production of tert-butyl {2-chloro-5-[3-(3,5-dichlorophenyl)-4,4,4-trifluoro-3-hydroxybutyryl]benzyl} carbamate

In toluene (4 ml) were dissolved tert-butyl (5-acetyl-2-chlorobenzyl)carbamate (521 mg) obtained in Example 1, α,α,α-trifluoro-3,5-dichloroacetophenone (446 mg) and triethylamine (136 mg), and stirred at 80°C for 2 hours. This reaction mixture liquid cooled to room temperature was concentrated under reduced pressure, and the resulting residue was subjected to silica gel column chromatography to obtain tert-butyl {2-chloro-5-[3-(3,5-dichlorophenyl)-4,4,4-trifluro-3-hydroxybutyryl]benzyl}carbamate (486 mg, Compound of Table 1-No. 12).
¹H-NMR (CDCl₃) δ: 7.94 (1H, d), 7.78 (1H, dd), 7.53-7.46 (3H, m), 7.35 (1H, t), 5.67 (1H, s), 5.08 (1H, br s), 4.46 (2H, d), 3.81 (1H, d), 3.66 (1H, d), 1.47 (9H, s).

### Example 3

### Production of tert-butyl {2-chloro-5-[3-(3,5-dichlorophenyl)-4,4,4-trifluoro-2-butenoyl]benzyl}carbamate

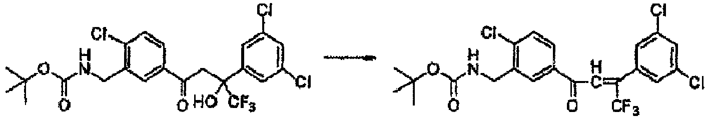

In tetrahydrofuran (4 ml) were dissolved tert-butyl {2-chloro-5-[3-(3,5-dichlorophenyl)-4,4,4-trifluoro-3-hydroxybutyryl]benzyl}carbamate (489 mg) obtained in Example 2, triethylamine (188 mg) and N,N-dimethyl-4-aminopyridine (14 mg), and acetic anhydride (95 mg) was added dropwise thereto at room temperature. After stirring at room temperature for 24 hours, this reaction mixture liquid was dissolved in methyl tert-butyl ether, and then washed with 1 M hydrochloric acid and an aqueous saturated sodium bicarbonate solution. The organic layer was dried over anhydrous sodium sulfate and then concentrated under reduced pressure to obtain crude tert-butyl {2-chloro-5-[3-(3,5-dichlorophenyl)-4,4,4-trifluoro-2-butenoyl]benzyl]carbamate (404 mg, Compound of Table 2-No.12). ¹H-NMR (CDCl₃) δ: 7.81 (1H, s), 7.66 (1H, d), 7.46-7.41 (1H, m), 7.36 (1H, br s), 7.32 (1H, d), 7.15 (2H, br s), 5.17 (1H, s), 4.41 (2H, d), 1.47 (9H, s).

### Example 4

### Production of tert-butyl {2-chloro-5-[5-(3,5-dichlorophenyl)-5-trifluoromethyl-4,5-dihydroisoxazol-3-yl] benzyl}carbamate

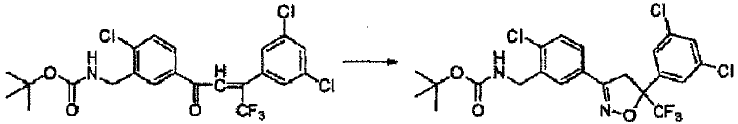

The crude tert-butyl {2-chloro-5-[3-(3,5-dichlorophenyl)-4,4,4-trifluoro-2-butenoyl]benzyl}carbamate (404 mg) obtained in Example 3 and tetrabutylammonium bromide (77 mg) were dissolved in toluene (1.6ml). Thereto was added dropwise a solution obtained by dissolving hydroxylamine hydrochloride (112 mg) and sodium hydroxide (134 mg) in water (0.6 ml) under ice-cooling, and the mixture was stirred under ice-cooling for 2 hours, then stirred at room temperature for 3 hours. To the reaction mixture was added 2 M hydrochloric acid, and the mixture was extracted with ethyl acetate. The organic layer was dried over anhydrous sodium sulfate and then concentrated under reduced pressure, and the resulting residue was subjected to silica gel column chromatography to obtain tert-butyl {2-chloro-5-[5-(3,5-dichlorophenyl)-5-trifluoromethyl-4,5-dihydroisoxazol-3-yl]benzyl}carbamate (295 mg). ¹H-NMR (CDCl₃) δ: 7.55-7.48 (6H, m), 5.06 (1H, br s), 4.41 (2H, d), 4.06 (1H, d), 3.68 (1H, d), 1.46 (9H, s).

### Example 5

### Production of 2-chloro-5-[5-(3,5-dichlorophenyl)-5-trifluoromethyl-4,5-dihydroisoxazol-3-yl]benzylamine

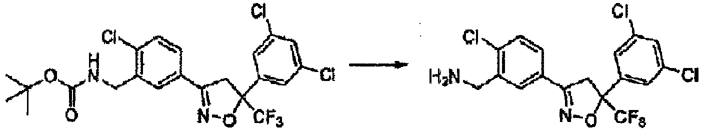

To tert-butyl {2-chloro-5-[5-(3,5-dichlorophenyl)-5-trifluoromethyl-4,5-dihydroisoxazol-3-yl]benzyl}carbamate (20.17 g) obtained in Example 4 was added trifluoroacetic acid (40 ml) at room temperature, and the mixture was stirred at room temperature for 1 hour. This reaction mixture was concentrated under reduced pressure, and an aqueous saturated sodium bicarbonate solution was added to the resulting residue, then the mixture was extracted with ethyl acetate. The organic layer was dried over anhydrous sodium sulfate and then concentrated under reduced pressure to obtain 2-chloro-5-[5-(3,5-dichlorophenyl)-5-trifluoromethyl-4,5-dihydroisoxazol-3-yl] benzylamine (15.49 g). ¹H-NMR (CDCl₃) δ: 7.72 (1H, d), 7.52-7.42 (5H, m), 4.09 (1H, d), 3.98 (2H, s), 3.71 (1H, d), 1.96 (2H, br s).

### Example 6

### Production of N-{2-chloro-5-[3-(3,5-dichlorophenyl)-4,4,4-trifluoro-3-hydroxybutyryl]benzyl}acetamide

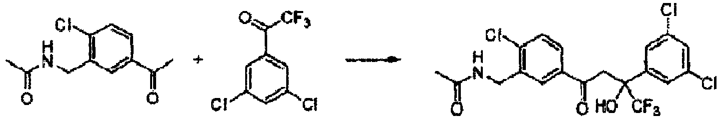

In toluene (5 ml) were dissolved N-(5-acetyl-2-chlorobenzyl]acetamide (1128 mg), α,α,α-trifluoro-3,5-dichloroacetophenone (1822 mg) and triethylamine (1019 mg), and stirred at 80°C for 4 hours. This reaction mixture liquid cooled to room temperature was concentrated under reduced pressure, and the resulting residue was subj ected to silica gel column chromatography to obtain N-{2-chloro-5-[3-(3,5-dichlorophenyl]-4,4,4-trifluoro-3-hydroxybutyryl]benzyl} acetamide (1490 mg, Compound of Table 1-No. 1).
¹H-NMR (CDCl₃) δ: 7.96 (1H, d), 7.79 (1H, dd), 7.51 (1H, d), 7.48 (2H, d), 7.35 (1H, t), 6.03-6.00 (1H, br m), 5.64 (1H, s), 4.56 (2H, d), 3.81 (1H, d), 3.66 (1H, d), 2.05 (3H, s).

### Example 7

### Production of N-{2-chloro-5-[3-(3,5-dichlorophenyl)-4,4,4-trifluoro-2-butenoyl]benzyl}acetamide

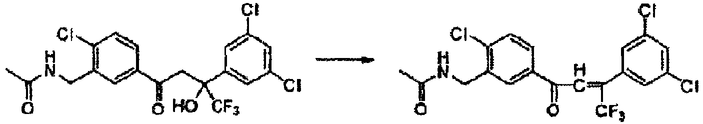

In tetrahydrofuran (2 ml) were dissolved N-{2-chloro-5- [3- (3, 5-dichlorophenyl)-4,4,4-trifluoro-3-hydroxybutyryl] benzyl}acetamide (469 mg) obtained in Example 6, triethylamine (121 mg) and N,N-dimethyl-4-aminopyridine (12 mg), and acetic anhydride (123 mg) was added dropwise thereto at room temperature. The mixture was stirred at room temperature for 3 hours, then stirred at 50°C for 2 hours. This reaction mixture liquid cooled to room temperature was concentrated under reduced pressure, and the resulting residue was subjected to silica gel column chromatography to obtain N-{2-chloro-5-[3-(3,5-dichlorophenyl)-4,4,4-trifluoro-2-butenoyl]benzyl} acetamide (410 mg, Compound of Table 2-No. 1).
¹H-NMR (CDCl₃) δ: 7.82 (1H, d), 7.68 (1H, dd), 7.45 (1H, d), 7.34-7.33 (2H, m), 7.15 (2H, d), 5.92 (1H, br s), 4.53 (2H, d), 2.06 (3H, s).

### Example 8

### Production of N-{2-chloro-5-[5-(3,5-dichlorophenyl)-5-trifluoromethyl-4,5-dihydroisoxazol-3-yl]benzyl}acetamide

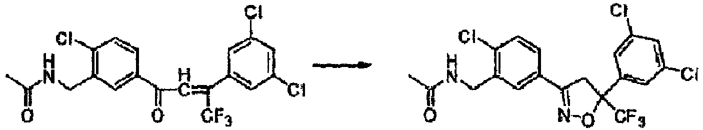

The crude N-{2-chloro-5-[3-(3,5-dichlorophenyl)-4,4,4-trifluoro-2-buteroyl]benzyl}acetamide (410 mg) obtained in Example 7 and tetrabutylammonium bromide (88 mg) were dissolved in tetrahydrofuran (1.8 ml). Thereto was added dropwise a solution obtained by dissolving hydroxylamine hydrochloride (126 mg) and sodium hydroxide (154 mg) in water (0.7 ml) under ice-cooling, and the mixture was stirred under ice-cooling for 1 hour, then stirred at room temperature for 2 hours. To the reaction mixture was added 2 M hydrochloric acid, and the mixture was extracted with ethyl acetate. The organic layer was dried over anhydrous sodium sulfate and then concentrated under reduced pressure, and the resulting residue was subjected to silica gel column chromatography to obtain N-{2-chloro-5-[5-(3,5-dichlorophenyl)-5-trifluoromethyl-4,5-dihydroisoxazol-3-yl]benzyl}acetamide (369 mg).
¹H-NMR (CDCl₃) δ: 7.64 (1H, d), 7.59 (1H, dd), 7.50 (2H, s), 7.43-7.42 (2H, m), 6.00-5.97 (1H, br m], 4.53 (2h, d), 4.07 (1H, d), 3.68 (1H, d), 2.04 (3H, s).

### Example 9

### Production of 2-chloro-5-[5-(3,5-dichlorophenyl)-5-trifluoromethyl-4,5-dihydroisoxazol-3-yl]benzylamine

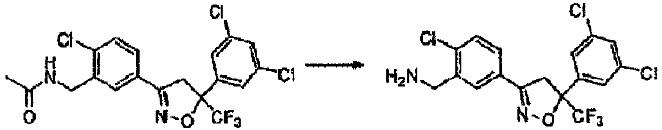

In ethanol (6 ml) was dissolved N-{2-chloro-5-[5-(3,5-dichlorophenyl)-5-trifluoromethyl-4,5-dihydroisoxazol-3-yl] benzyl}acetamide (200 mg) obtained in Example 8, and concentrated hydrochloric acid (2 ml) was added thereto at room temperature, then the mixture was stirred under reflux for 40 hours. This reaction mixture cooled to room temperature was concentrated under reduced pressure, and an aqueous saturated sodium bicarbonate solution was added to the resulting residue, then the mixture was extracted with ethyl acetate. The organic layer was dried over anhydrous sodium sulfate and then concentrated under reduced pressure to obtain 2-chloro-5-[5-(3,5-dichlorophenyl)-5-trifluoromethyl-4,5-dihydroisoxazol--3-yl]benzylamine (160 mg). ¹H-NMR (CDCl₃) δ: 7.72 (1H, d), 7.52-7.42 (5H, m), 4.09 (1H, d), 3.98 (2H, s), 3.71 (1H, d), 1.96 (2H, br s).

## Claims

1. An aldol compound represented by formula (3): 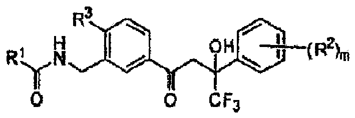 [wherein R¹ represents a C1-C12 alkyl group optionally having one or more halogen atoms, a C3-C12 cycloalkyl group optionally having one or more halogen atoms, or a C1-C12 alkoxy group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another) ].

2. An enone compound represented by formula (5) : 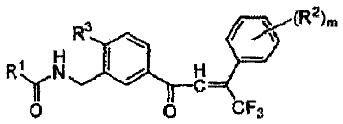 [wherein R¹ represents a C1-C12 alkyl group optionally having one or more halogen atoms, a C3-C12 cycloalkyl group optionally having one or more halogen atoms, or a C1-C12 alkoxy group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)].

3. tert-Butyl (5-acetyl-2-chlorobenzyl)carbamate.

4. tert-Butyl {2-chloro-5-[5-(3,5-dichlorophenyl)-5-trifluoromethyl-4,5-dihydroisoxazol-3-yl]benzyl}carbamate.

5. A method for producing an aldol compound represented by formula (3): 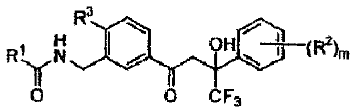 [wherein R¹ represents a C1-C12 alkyl group optionally having one or more halogen atoms, a C3-C12 cycloalkyl group optionally having one or more halogen atoms or a C1-C12 alkoxy group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)], comprising a step of reacting an amide compound represented by formula (1) : 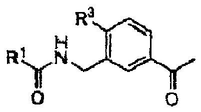 [wherein R¹ and R³ represent the same meanings as above] with a trifluoroacetophenone compound represented by formula (2): 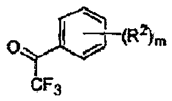 [wherein R² and m represent the same meanings as above].

6. A method for producing an enone compound represented by formula (5): 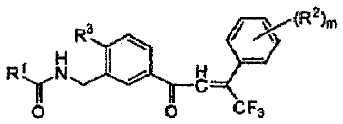 [wherein R¹ represents a C1-C12 alkyl group optionally having one or more halogen atoms, a C3-C12 cycloalkyl group optionally having one or more halogen atoms, or a C1-C12 alkoxy group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)], comprising a step of reacting an aldol compound represented by formula (3): 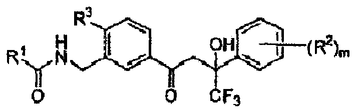 [wherein R¹, R², R³ and m represent the same meanings as above] with an acid anhydride compound represented by formula (4): 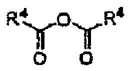 [wherein R⁴ represents a C1-C12 alkyl group optionally having one or more halogen atoms or a C1-C12 alkoxy group optionally having one or more halogen atoms].

7. A method for producing an isoxazoline compound represented by formula (6): 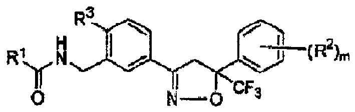 [wherein R¹ represents a C1-C12 alkyl group optionally having one or more halogen atoms, a C3-C12 cycloalkyl group optionally having one or more halogen atoms, or a C1-C12 alkoxy group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)], comprising a step of reacting an enone compound represented by formula (5): 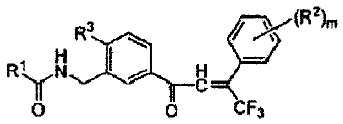 [wherein R¹, R², R³ and m represent the same meanings as above] with hydroxylamine.

8. A method for producing a benzylamine compound represented by formula (7): 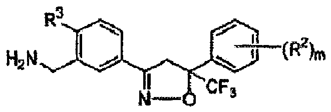 [wherein R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)],
comprising a step of reacting an isoxazoline compound represented by formula (6): 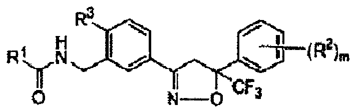 [wherein R¹ represents a C1-C12 alkyl group optionally having one or more halogen atoms, a C3-C12 cycloalkyl group optionally having one or more halogen atoms, or a C1-C12 alkoxy group optionally having one or more halogen atoms, and R², R³ and m represent the same meanings as above]
with an acid.

9. A method for producing a benzylamine compound represented by formula (7): 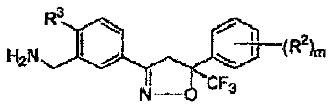 [wherein R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)],
comprising steps of
reacting an amide compound represented by formula (1): 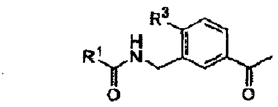 [wherein R¹ represents a C1-C12 alkyl group optionally having one or more halogen atoms, a C3-C12 cycloalkyl group optionally having one or more halogen atoms or a C1-C12 alkoxy group optionally having one or more halogen atoms, and R³ represents the same meaning as above]
with a trifluoroacetophenone compound represented by formula (2): 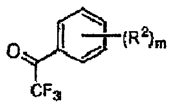 [wherein R² and m represent the same meanings as above] to produce an aldol compound represented by formula (3): 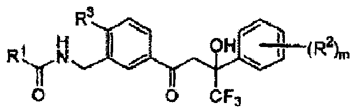 [wherein R¹, R², R³ and m represent the same meanings as above], reacting the obtained aldol compound represented by formula (3) with an acid anhydride compound represented by formula (4): 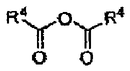 [wherein R⁴ represents a C1-C12 alkyl group optionally having one or more halogen atoms or a C1-C12 alkoxy group optionally having one or more halogen atoms]
to produce an enone compound represented by formula (5): 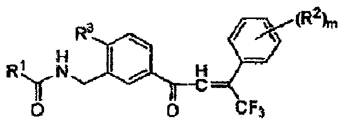 [wherein R¹, R², R³ and m represent the same meanings as above], reacting the obtained enone compound represented by formula (5) with hydroxylamine to produce an isoxazoline compound represented by formula (6): 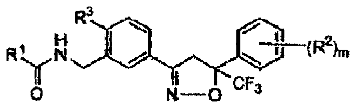 [wherein R¹, R², R³ and m represent the same meanings as above], and
reacting the obtained isoxazoline compound represented by the formula (6) with an acid.
